# EUROPEAN PATENT SPECIFICATION

(11) **EP 3 412 143 B1**
(45) Date of publication and mention of the grant of the patent: **12.08.2020**
(21) Application number: 16889216.4
(22) Date of filing: 01.02.2016
(51) Int. Cl.: A01K 1/015

(54) **METHOD FOR MAKING A WATER-ABSORBING TREATMENT MATERIAL**
VERFAHREN ZUR HERSTELLUNG EINES WASSERABSORBIERENDEN BEHANDLUNGSMATERIAL
PROCEDE DE FABRICATION D'UN MATERIAU DE TRAITEMENT ABSORBANT L'EAU

(43) Date of publication of application: 12.12.2018
(73) Proprietor: Daiki Co., Ltd., Minato-ku Tokyo 107-0052 (JP)
(72) Inventor: ITO, Hiroshi, Tokyo 107-0052 (JP); YOSHINAGA, Junji, Tokyo 107-0052 (JP); HATANAKA, Shinobu, Tokyo 107-0052 (JP); HOSOYA, Takahiro, Tokyo 107-0052 (JP); MASUYAMA, Takuya, Tokyo 107-0052 (JP)
(74) Representative: Müller-Boré & Partner Patentanwälte PartG mbB
(86) International application number: PCT/JP2016/052903
(87) International publication number: WO 2017/134726

(56) References cited:
- JP-A- H09 308 403
- JP-A- 2013 071 107
- JP-A- 2013 071 107
- JP-A- 2015 198 600
- JP-A- 2015 198 600
- US-A1- 2014 190 420

## Description

### Technical Field

The present invention relates to method for manufacturing a water absorption treatment material that absorbs human or animal excrements and other liquids.

### Background Art

Patent Document 1 discloses an excrement treatment material, which is a type of water absorption treatment material. This excrement treatment material is provided with a core layer that contains fibers and a skin layer that covers the core layer. The skin layer contains an α-starch and fibers. Patent Document 1 states that an advantage of this excrement treatment material is that the excrement treatment material can be flushed down a flush toilet with peace of mind.

Patent Document 2 discloses a method for manufacturing an excrement treating agent comprising preparing a first mass comprising a water-absorbing polymer, a binder and an acid solid material, granulating and then mixing with an alkaline substance which can be sodium bicarbonate. Gas bubbles are formed in the material.

Patent document 3 discloses a granular excrement treating material which can be easily disintegrated comprising a water retaining material, an adhesive and a water absorbing resin.

Patent document 4 discloses a water absorbing material having excellent disintegrability comprising a granular core part and a coating layer part.

### Citation List

### Patent Document

Patent Document 1: JP 2010-104383A
Patent Document 2: JP 2013-71107A
Patent Document 3: US 2014/0190420 A1
Patent Document 4: JP 2015-198600 A

### Summary of Invention

### Technical Problem

However, in order to dispose of the used water absorption treatment material by flushing the water absorption treatment material down a flush toilet, it is necessary that the water absorption treatment material has sufficient water disintegrability (the property of being able to disperse in water due to fibers and particles that are bound together quickly separating upon contact with water). If the water disintegrability of the water absorption treatment material is insufficient, the water absorption treatment material may clog the toilet. In this respect, conventional water absorption treatment materials have room for improvement in terms of water disinte grability.

The present invention was made in view of the above-described problem, and it is an object thereof to provide a method for manufacturing the water absorption treatment material that has excellent water disintegrability.

### Solution to Problem

A water absorption treatment material which is disclosed is a water absorption treatment material that absorbs a liquid, the water absorption treatment material including a granule that contains a foaming agent, wherein, upon the water absorption treatment material absorbing the liquid, gas bubbles are produced in the granule as a result of a reaction of the foaming agent, and the granule collapses.

In this water absorption treatment material, the foaming agent is contained in the granule. Upon the liquid being absorbed, the foaming agent reacts therewith, gas bubbles are thus produced in the granule, and the granule collapses. The water disintegrability of the water absorption treatment material can be improved due to the granule collapsing upon absorbing the liquid.

A method for manufacturing a water absorption treatment material according to the present invention is a method for manufacturing a water absorption treatment material that absorbs a liquid, the method including an adding step of adding a foaming agent composed of sodium hydrogen carbonate to a material to be granulated, and a granulating step of granulating the material to be granulated to which the foaming agent is added and thereby forming a granule.

In this manufacturing method, prior to granulation of the material to be granulated, the foaming agent is added to the material to be granulated. Thus, a granule that contains the foaming agent can be obtained. In the manufactured water absorption treatment material, when the water absorption treatment material absorbs a liquid, the foaming agent reacts therewith, and thus, gas bubbles are produced in the granule. Due to the generation of gas bubbles, collapse of the granule is accelerated, and therefore, the water disintegrability of the water absorption treatment material can be improved.

### Advantageous Effects of Invention

According to the present invention, a method for manufacturing a water treatment absorption material that has excellent disintegrability can be realized.

### Brief Description of Drawings

FIG. 1 is a schematic diagram showing a first water absorption treatment material.
FIG. 2 is a diagram for illustrating a change that occurs when the water absorption treatment material in FIG. 1 absorbs a liquid.
FIG. 3 is a schematic diagram showing a second water absorption treatment material .
FIG. 4 is a schematic diagram showing a water absorption treatment material according to a modification.

### Description of Embodiments

Hereinafter, some embodiments will be described in detail with reference to the drawings. It should be noted that the description of the drawings denotes like elements with like reference numerals and omits redundant descriptions.

### (First Embodiment)

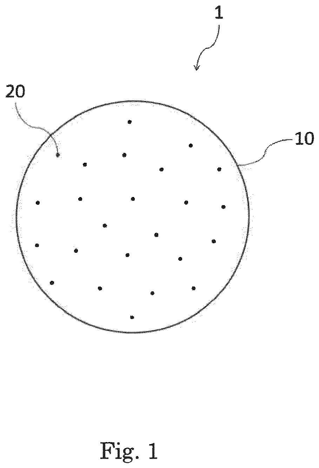

FIG. 1 is a schematic diagram showing a first water absorption treatment material. A water absorption treatment material 1 is a water absorption treatment material that absorbs a liquid, and includes a granule 10. The water absorption treatment material 1 is, for example, an excrement treatment material for pet animals such as cats and dogs.

The granule 10 has the function of absorbing and retaining a liquid such as urine. The granule 10 has a granular shape. Examples of the shape of the granule 10 include a sphere, an ellipse, and a cylinder. The granule 10 contains paper as the main material. Here, "paper being the main material of the granule 10" means that the paper accounts for the highest weight ratio in all of the materials that constitute the granule 10. Examples of the paper include ordinary paper as well as a vinyl chloride wallpaper classified product (paper obtained through classification of vinyl chloride wallpaper), a fluff pulp, a papermaking sludge, and a pulp sludge. For example, a product that is obtained by classifying disposable diapers and removing an absorbent polymer can be used as the fluff pulp. Note that, since it is difficult to completely remove an absorbent polymer, it is normal that a small amount of absorbent polymer adheres to such a fluff pulp.

The above-described paper is pulverized to a particle size (fiber length) of 1 mm or less. That is to say, the paper is obtained through pulverization using a pulverizer with a screen having an aperture size of 1 mm or less. The particle size of the paper is preferably 0.5 mm or less, and more preferably 0.3 mm or less.

The granule 10 contains an adhesive material (first adhesive material). Examples of the adhesive material include dextrin, starch, CMC (carboxymethyl cellulose), PVA (polyvinyl alcohol), and a water-absorbent polymer. Preferably, the water-absorbent polymer has a mean particle size of 20 µm or less. The mean particle size as used herein refers to the smallest aperture size through which 50 wt% or more of particles can pass when the water-absorbent polymer, which is an aggregate of a large number of particles, is sieved. Therefore, "having a mean particle size of 20 µm or less" means that when the water-absorbent polymer is sieved using a sieve with an aperture size of 20 µm, 50 wt% or more of the particles can pass through the sieve. In the present embodiment, dextrin is used as the first adhesive material. Preferably, the weight ratio of the first adhesive material in the granule 10 is 10% or more and 30% or less.

The granule 10 contains a foaming agent 20. The foaming agent 20 contained in the granule 10 is in an unreacted state. However, the granule 10 may also contain a reacted foaming agent. The reacted foaming agent is a portion of the foaming agent 20 that has been reacted in the manufacturing process of the water absorption treatment material 1. A foaming agent refers to a substance that produces bubbles as a result of a chemical reaction such as thermal decomposition. In the present invention, sodium hydrogen carbonate is used as the foaming agent 20. The granule 10 that contains the foaming agent 20 as described above can be formed by granulating, for example, a material to be granulated to which the foaming agent 20 is added.

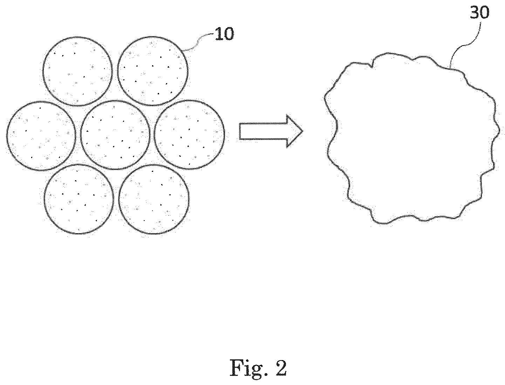

The granule 10 is configured such that, upon the water absorption treatment material 1 absorbing a liquid, gas bubbles are produced in the granule 10 as a result of the reaction of the foaming agent 20, and the granule 10 collapses. More specifically, upon the sodium hydrogen carbonate (foaming agent 20) undergoing a reaction, in addition to sodium carbonate and water, carbon dioxide is generated, and the generated carbon dioxide causes gas bubbles to be produced in the granule 10. Note that "the granule 10 collapsing" means that the granule 10 breaks up to such an extent that its granular shape can no longer be maintained. The water absorption treatment material 1 is used in a state in which a plurality of granules 10 are spread out in an animal toilet or the like. As shown in FIG. 2, a plurality of granules 10 that have collapsed upon being exposed to a liquid such as urine mix together to form a mixture 30, which is a sludgy mass. In this mixture 30, the individual granules 10 can no longer be distinguished from one another.

An example of a method for manufacturing the water absorption treatment material 1 will be described as a first embodiment of a method for manufacturing a water absorption treatment material according to the present invention. First, a material (material to be granulated) that the granule 10 is to be composed of is pulverized to a predetermined size using a pulverizer, and the pulverized material to be granulated is placed in a mixer at a predetermined rate, and mixed. At this time, the foaming agent 20 is added to the material to be granulated (adding step). For example, the foaming agent 20 can be added to the material to be granulated by placing the foaming agent 20 into the mixer together with the material to be granulated and mixing them. In the case of the present embodiment, paper, dextrin, and sodium hydrogen carbonate are mixed, and the mixing can be performed step-by-step. For example, a method is conceivable in which dextrin and sodium hydrogen carbonate are mixed beforehand, and then mixed with paper.

Next, water is added to the material to be granulated, as necessary, and then, the material to be granulated to which the foaming agent 20 has been added is granulated to thereby form granules 10 (granulating step). At this time, in order to obtain granules 10 that can easily collapse upon absorbing a liquid, it is preferable to reduce the compressive force that is applied to the material to be granulated during granulation. For example, the compressive force applied to the material to be granulated can be reduced by reducing the thickness of a die of a granulator.

Subsequently, the granules 10 that have been produced through the prior steps are sieved using a sieve with a predetermined mesh size (sizing step). Thus, only the granules 10 that satisfy a predetermined standard are extracted. Then, the granules 10 that have been extracted in the prior step are dried using a dryer (drying step). The water content of the granules 10 is adjusted as appropriate through drying. Thus, it is possible to prevent the occurrence of mold and the like during storage of the water absorption treatment material 1. In the above-described manner, a water absorption treatment material 1 can be obtained.

The effects of the present embodiment will be described. In the water absorption treatment material 1, the granule 10 contains the foaming agent 20. Upon a liquid being absorbed, the foaming agent 20 reacts therewith, and thus, gas bubbles are produced in the granule 10. Due to the generation of gas bubbles, collapse of the granule 10 is accelerated. Actually, in the present embodiment, the granule 10 is configured to collapse upon absorbing a liquid. Thus, compared with a case where granule 10 does not collapse even after absorbing a liquid, the water disintegrability of the water absorption treatment material 1 can be improved.

The granule 10 is formed by granulating the material to be granulated to which the foaming agent 20 has been added. That is to say, prior to granulation of the material to be granulated, the foaming agent 20 is added to the material to be granulated. Thus, a granule 10 that contains the foaming agent 20 can be easily obtained.

The granule 10 contains paper as the main material. Paper is a material with excellent water-absorbing properties and water-retaining properties. However, in conventional water absorption treatment materials that contain paper as the main material, relatively long fibers (pulp) that constitute the paper are entangled and are unlikely to come apart, and this is a factor that prevents an improvement in water disintegrability. In this respect, according to the present embodiment, as described above, gas bubbles are generated in the granule 10 by the foaming agent 20, and thus collapse of the granule 10 is accelerated. Thus, it is possible to realize a water absorption treatment material 1 with excellent water disintegrability while using paper as the main material.

The above-described paper is pulverized to a particle size of 1 mm or less. As a result of finely pulverizing the paper as described above, fibers constituting the paper are likely to come apart, and the water disintegrability of the water absorption treatment material 1 can be improved. From this point of view, the paper is preferably pulverized to a particle size of 0.5 mm or less, and more preferably pulverized to a particle size of 0.3 mm or less.

The granule 10 contains dextrin as the adhesive material. Thus, the shape retention property of the granule 10 before the water absorption treatment material 1 is used (before the water absorption treatment material 1 absorbs a liquid) can be improved. Moreover, dextrin has the property of decreasing its viscosity when absorbing a liquid. Accordingly, once the water absorption treatment material 1 has absorbed a liquid, the viscosity of dextrin in the granule 10 decreases. This, in combination with the generation of gas bubbles by the foaming agent 20, also accelerates collapse of the granule 10.

Dextrin whose viscosity has decreased due to absorption of a liquid recovers its viscosity when it dries. Accordingly, when a predetermined period of time has elapsed after the water absorption treatment material 1 has absorbed a liquid, dextrin in the mixture 30 dries and recovers its viscosity. Thus, dextrin contributes to an improvement in the bonding strength of the mixture 30. Note that adhesive materials such as dextrin are relatively expensive. Therefore, a reduction in the amount of adhesive material that is used contributes to a reduction in the manufacturing cost of the water absorption treatment material 1. From this point of view, the weight ratio of the adhesive material in the granule 10 is preferably 30% or less.

### (Second Embodiment)

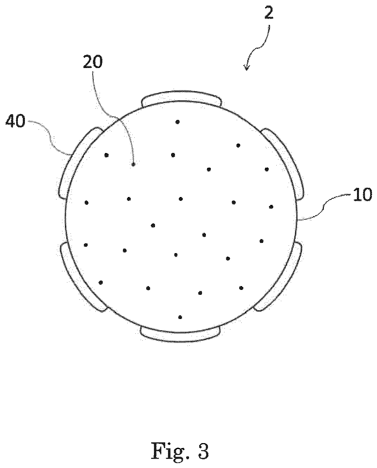

FIG. 3 is a schematic diagram showing a second water absorption treatment material A water absorption treatment material 2 is a water absorption treatment material that absorbs a liquid, and includes a coating layer portion 40 in addition to the granule 10. The granule 10 is configured as described above in the first embodiment.

The coating layer portion 40 covers the granule 10. The coating layer portion 40 is a portion that covers at least a portion of the surface of the granule 10. In the present embodiment, the coating layer portion 40 covers only a portion of the surface of the granule 10. The weight ratio of the coating layer portion 40 in the whole of the granule 10 and the coating layer portion 40 is preferably 1% or more and less than 15%, and more preferably 1% or more and less than 10%. Paper, for example, can be used as the main material of the coating layer portion 40 as well.

The coating layer portion 40 contains an adhesive material (second adhesive material). The same adhesive material as the above-described first adhesive material may be used as the second adhesive material, or a different adhesive material may be used. In the present embodiment, a water-absorbent polymer that has a mean particle size of 20 µm or less is used as the second adhesive material.

An example of a method for manufacturing the water absorption treatment material 2 will be described as a second embodiment of the method for manufacturing a water absorption treatment material according to the present invention. First, granules 10 are formed using the method described in the first embodiment. Next, a material (coating material) that the coating layer portion 40 is to be composed of is made to adhere to the surface of each granule 10 to thereby form the coating layer portions 40 (coating step). Note that, in the coating step, water is not added to the granules 10. That is to say, in the present embodiment, the coating material is made to adhere to the surface of each granule 10 without adding water to the granules 10. The coating material can be made to adhere to the surface of each granule 10 through sprinkling or spraying using a coating apparatus, for example. Then, the sizing step and the drying step are conducted, and thus, a water absorption treatment material 2 can be obtained.

The effects of the present embodiment will be described. In the water absorption treatment material 2, the coating layer portion 40, which contains the adhesive material, is formed. Thus, the bonding strength of a mixture that is formed during use of the water absorption treatment material 2 can be increased.

A reduction in the ratio of the coating layer portion 40 contributes to a reduction in the cost of procuring materials and hence the manufacturing cost of the water absorption treatment material 2. From this point of view, the weight ratio of the coating layer portion 40 in the whole of the granule 10 and the coating layer portion 40 is preferably less than 15%, and more preferably less than 10%. On the other hand, if the ratio of the coating layer portion 40 is excessively small, the effect of the coating layer portion 40 of increasing the bonding strength will not be sufficiently provided. From this point of view, it is preferable that the weight ratio of the coating layer portion 40 in the whole of the granule 10 and the coating layer portion 40 is 1% or more.

The coating layer portion 40 covers only a portion of the surface of the granule 10. In other words, the other portion of the surface of the granule 10 is exposed without being covered by the coating layer portion 40. Thus, during use of the water absorption treatment material 2, the granule 10 can quickly absorb a liquid. This accelerates the reaction of the foaming agent 20 and is advantageous in increasing the collapsibility of the granule 10.

The coating layer portion 40 contains a water-absorbent polymer as the adhesive material. Water absorbent polymers have the property of swelling upon absorbing a liquid, and the swollen water-absorbent polymer in the coating layer portion 40 prevents the liquid from reaching the granule 10. Thus, swelling of the water-absorbent polymer may decrease the collapsibility of the granule 10 and hence the water disintegrability of the water absorption treatment material 2. In this respect, swelling of the water-absorbent polymer upon absorbing a liquid can be suppressed by finely pulverizing the water-absorbent polymer. Accordingly, in order to suppress swelling of the water-absorbent polymer, the mean particle size of the water-absorbent polymer contained in the coating layer portion 40 is preferably 20 µm or less, and more preferably 10 µm or less.

In the coating step, the coating material is made to adhere to the surface of the granule 10 without adding water to the granule 10. Thus, it is possible to prevent more coating material than is necessary from adhering to the surface of the granule 10. Moreover, the manufacturing process of the water absorption treatment material 2 can be simplified by omitting the water addition step. Note that, even when the water addition step is omitted as described above, water that is contained in the granule 10 can account for the water that is necessary for adhesion of the coating material. The other effects of the present embodiment are as described above in the first embodiment.

The method for manufacturing the water absorption treatment material according to the present invention are not limited to the foregoing embodiments, and various modifications can be made. In the foregoing embodiments, the foaming agent is composed of sodium hydrogen carbonate. However, a foaming agent composed of sodium hydrogen carbonate and citric acid is also disclosed. In this case, the composition (weight ratios) of the granule 10 can be set as follows, for example: 80% paper, 10% dextrin, 6.5% sodium hydrogen carbonate, and 3.5% citric acid. Blending citric acid with sodium hydrogen carbonate in this manner can accelerate the reaction of the foaming agent.

In the foregoing embodiments, paper has been described as an example of the main material of the granule 10. However, water-absorbent materials other than paper may also be used as the main material of the granule 10. Examples of such water-absorbent materials include plastics or used tea leaves. The same applies to the main material of the coating layer portion 40.

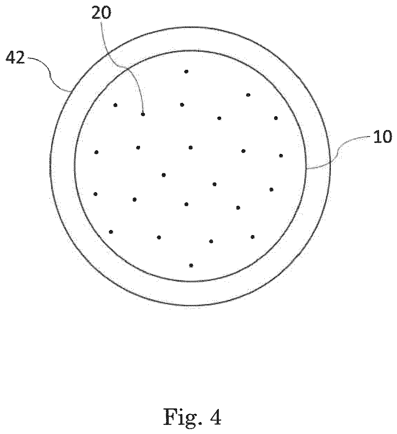

In the foregoing embodiments, a case where only a portion of the surface of the granule 10 is covered by the coating layer portion 40 has been described. However, as shown in FIG. 4, the surface of the granule 10 may be entirely covered by a coating layer portion 42. The configuration of the coating layer portion 42 is the same as that of the coating layer portion 40 except that the coating layer portion 42 covers the entire surface of the granule 10. Note that the coating layer portion 42 may also contain the foaming agent 20. That is to say, a configuration may be adopted in which both the granule 10 and the coating layer portion 42 contain the foaming agent 20. Such coating layer portion 42 can be formed by, for example, adding the foaming agent 20 to the coating material, in the coating step, before making the coating material adhere to the granule 10. If the coating layer portion 42 also contains the foaming agent 20 as described above, collapse of not only the granule 10 but also the coating layer portion 42 can be accelerated.

### List of Reference Numerals

- 1: Water Absorption Treatment Material
- 2: Water Absorption Treatment Material
- 10: Granule
- 20: Foaming Agent
- 30: Mixture
- 40: Coating Layer Portion
- 42: Coating Layer Portion

## Claims

1. A method for manufacturing a water absorption treatment material that absorbs a liquid, the method comprising:
an adding step of adding a foaming agent composed of sodium hydrogen carbonate to a material to be granulated; and
a granulating step of granulating the material to be granulated to which the foaming agent is added and thereby forming a granule.

2. The method for manufacturing a water absorption treatment material according to claim 1, the method further comprising:
a coating step of making a coating material adhere to a surface of the granule formed in the granulating step and thereby forming a coating layer portion that covers the granule.

3. The method for manufacturing a water absorption treatment material according to claim 2,
wherein, in the coating step, the coating layer portion is formed so as to cover only a portion of the surface of the granule.

4. The method for manufacturing a water absorption treatment material according to claim 2,
wherein, in the coating step, the coating layer portion is formed so as to cover an entire surface of the granule.

5. The method for manufacturing a water absorption treatment material according to claim 4,
wherein, in the coating step, the foaming agent is added to the coating material before the coating material is made to adhere to the surface of the granule.

6. The method for manufacturing a water absorption treatment material according to any one of claims 2 to 5,
wherein, in the coating step, the coating material is made to adhere to the surface of the granule without adding water to the granule.

## Patentansprüche

1. Verfahren zur Herstellung eines Wasserabsorptionsbehandlungsmaterials, welches eine Flüssigkeit absorbiert, wobei das Verfahren umfasst:
einen Hinzufügungsschritt des Hinzufügens eines Schäummittels, bestehend aus Natriumhydrogencarbonat, zu einem zu granulierenden Material; und
einen Granulierungsschritt des Granulierens des zu granulierenden Materials, zu welchem das Schäummittel hinzugefügt wird, und des Bildens eines Granulats dadurch.

2. Verfahren zur Herstellung eines Wasserabsorptionsbehandlungsmaterials nach Anspruch 1, wobei das Verfahren weiter umfasst:
einen Beschichtungsschritt, der ein Beschichtungsmaterial dazu bringt an einer Oberfläche des Granulats, gebildet in dem Granulierungsschritt, zu haften und dadurch einen Beschichtungsschichtteil, welcher das Granulat bedeckt, bildet.

3. Verfahren zur Herstellung eines Wasserabsorptionsbehandlungsmaterials nach Anspruch 2,
wobei in dem Beschichtungsschritt der Beschichtungsschichtteil so gebildet wird, dass er nur einen Teil der Oberfläche des Granulats bedeckt.

4. Verfahren zur Herstellung eines Wasserabsorptionsbehandlungsmaterials nach Anspruch 2,
wobei in dem Beschichtungsschritt der Beschichtungsschichtteil so gebildet wird, dass er eine gesamte Oberfläche des Granulats bedeckt.

5. Verfahren zur Herstellung eines Wasserabsorptionsbehandlungsmaterials nach Anspruch 4,
wobei in dem Beschichtungsschritt das Schäummittel zum Beschichtungsmaterial hinzugefügt wird, bevor das Beschichtungsmaterial dazu gebracht wird, an der Oberfläche des Granulats zu haften.

6. Verfahren zur Herstellung eines Wasserabsorptionsbehandlungsmaterials nach einem der Ansprüche 2 bis 5,
wobei in dem Beschichtungsschritt das Beschichtungsmaterial dazu gebracht wird an der Oberfläche des Granulats zu haften, ohne Wasser zum Granulat hinzuzufügen.

## Revendications

1. Procédé de fabrication d'une matière de traitement par absorption d'eau qui absorbe un liquide, le procédé comprenant:
une étape d'addition où l'on ajoute un agent moussant composé d'hydrogéno-carbonate de sodium à une matière à granuler; et
une étape de granulation où l'on granule la matière à granuler à laquelle l'agent moussant est ajouté et l'on forme ainsi un granule.

2. Procédé de fabrication d'une matière de traitement par absorption d'eau selon la revendication 1, le procédé comprenant en outre:
une étape de revêtement où l'on fait qu'une matière de revêtement adhère à la surface du granulé formé à l'étape de granulation et l'on forme ainsi une partie de couche de revêtement qui recouvre le granule.

3. Procédé de fabrication d'une matière de traitement par absorption d'eau selon la revendication 2,
dans lequel, à l'étape de revêtement, la partie de couche de revêtement est formée de manière à ne recouvrir qu'une partie de la surface du granule.

4. Procédé de fabrication d'une matière de traitement par absorption d'eau selon la revendication 2,
dans lequel, à l'étape de revêtement, la partie de couche de revêtement est formée de manière à recouvrir la totalité de la surface du granule.

5. Procédé de fabrication d'une matière de traitement par absorption d'eau selon la revendication 4,
dans lequel, à l'étape de revêtement, l'agent moussant est ajouté à la matière de revêtement avant que la matière de revêtement n'adhère à la surface du granule.

6. Procédé de fabrication d'une matière de traitement par absorption d'eau selon l'une quelconque des revendications 2 à 5,
dans lequel, à l'étape de revêtement, la matière de revêtement fait qu'elle adhère à la surface du granule sans addition d'eau au granule.
